(19) 

Europäisches
Patentamt
European
Patent Office
Office européen
des brevets

(11) **EP 4 125 934 B1**

(12) **EUROPEAN PATENT SPECIFICATION**

(45) Date of publication and mention
of the grant of the patent:
**06.08.2025 Bulletin 2025/32**

(21) Application number: **21712887.5**

(22) Date of filing: **24.03.2021**

(51) International Patent Classification (IPC):
*A61K 31/715* (2006.01)    *A61K 31/702* (2006.01)
*A61K 31/738* (2006.01)    *A61K 45/06* (2006.01)
*A61P 3/06* (2006.01)      *A61P 3/10* (2006.01)
*A23L 33/10* (2016.01)     *A23L 33/00* (2016.01)

(52) Cooperative Patent Classification (CPC):
(C-Sets available)
**A61K 31/702; A23L 33/10; A23L 33/40;
A61K 31/715; A61K 31/738; A61K 45/06;
A61P 3/06; A61P 3/10**                      (Cont.)

(86) International application number:
**PCT/EP2021/057530**

(87) International publication number:
**WO 2021/191251 (30.09.2021 Gazette 2021/39)**

(54) **COMPOSITIONS COMPRISING ONE OR MORE HMO'S WITH A CORE OF LACNAC-LAC**

ZUSAMMENSETZUNGEN MIT EINEM ODER MEHREREN HMOS MIT EINEM KERN AUS
LACNAC-LAC

COMPOSITIONS COMPRENANT UN OU PLUSIEURS HMO AYANT UN NOYAU DE LACNAC-LAC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.03.2020 EP 20165487**

(43) Date of publication of application:
**08.02.2023 Bulletin 2023/06**

(73) Proprietor: **FrieslandCampina Nederland B.V.
3818 LE Amersfoort (NL)**

(72) Inventor: **TRIANTIS, Vassilios
6708 WH Wageningen (NL)**

(74) Representative: **FrieslandCampina IP Department
Bronland 20
6708 WH Wageningen (NL)**

(56) References cited:
**WO-A1-2017/215721      WO-A1-2019/052529
US-A1- 2017 095 491      US-A1- 2019 269 713**

**(Cont. next page)**

(52) Cooperative Patent Classification (CPC): (Cont.)

C-Sets
**A61K 31/702, A61K 2300/00;**
**A61K 31/715, A61K 2300/00;**
**A61K 31/738, A61K 2300/00**

**Description**

FIELD OF THE INVENTION

The invention disclosed herein relates to a synthetic composition comprising one or more compounds according to formula 1, or a pharmaceutically acceptable salt, solvate or ester thereof, for use in the reduction of the cholesterol level: wherein the compound of Formula 1 is

$$Y_n \rightarrow D\text{-}Gal\text{-}\beta 1 \rightarrow 3\text{-}(Y_m \rightarrow)(X_p \rightarrow)D\text{-}GlcNAc\text{-}\beta 1 \rightarrow 3\text{-}D\text{-}Gal\text{-}\beta 1 \rightarrow 4\text{-}(X_q \rightarrow)\text{-}D\text{-}Glc \qquad \text{Formula 1}$$

The invention further relates to the use of this synthetic composition for use in medicine.

BACKGROUND

Breast feeding is the best way to ensure healthy growth and development of infants during the first months of life. It is recommended by the WHO to exclusively provide breast feeding during the first six months of life and the introduction of safe and appropriate complementary feeding thereafter to supplement continued breast feeding up to two years of age or beyond. However, when mothers cannot or choose not to breastfeed for whatever reason and a safe alternative to breast feeding is required, there is a legitimate role for breast milk substitutes, produced according to strict international compositional and safety standards.

WO2019052529 discloses LNFP III to reduce cholesterol levels and to be useful for the treatment of hyperlipidemia. LNFP III is not a compound according to Formula 1 because the linkage of D-Gal to D-GlcNAc is 1-->4 instead of 1-->3.

Cholesterol is an essential structural component of all animal cell membranes and is essential to maintain both membrane structural integrity and fluidity. In addition it serves as a precursor for the biosynthesis of steroid hormones, bile acids and vitamin D. Humans get cholesterol through their diet. Since all animal cells manufacture cholesterol, all animal-based foods contain cholesterol in varying amounts. Human breast milk also contains significant quantities of cholesterol, since cholesterol is needed to support fast growth of infant tissue and brain development but also cognitive functions in adulthood.

Excess of cholesterol intake through a high fat diet and high cholesterol levels in peripheral tissues can lead to adverse conditions such as immune dysregulation, formation of foam cells, obesity, atherogenesis, atherosclerosis and eventually cardiovascular disease. Since cholesterol is insoluble in water, it is transported in the blood plasma within protein particles (lipoproteins). Lipoproteins are classified by their density: very low density lipoprotein (VLDL), intermediate density lipoprotein (IDL), low density lipoprotein (LDL) and high density lipoprotein (HDL). All the lipoproteins carry cholesterol, but elevated levels of the lipoproteins other than HDL (termed non-HDL cholesterol), particularly LDL-cholesterol, are associated with an increased risk of atherosclerosis and coronary heart disease. In contrast, higher levels of HDL cholesterol are protective towards coronary heart disease. The human body is cleaning cholesterol from peripheral tissues by a process called reverse cholesterol transport, that directs cholesterol back to the liver to be excreted in the form of bile acids. The first step in this process is transferring cholesterol to HDL by action of the ATP-binding cassette transporter encoded by the ABCA1 gene. Therefore higher expression of ABCA1 is increasing HDL levels and is protective for atherogenesis, i.e. the formation of fatty deposits in the arteries. There is thus a need to increase the expression of ABCA1 which may then reduce cholesterol levels, in particular within subjects suffering from high cholesterol levels or from atherosclerotic plaques.

If cholesterol is not cleared from the circulation properly then higher amounts of LDL will be taken up by monocytes through the CD36 surface transporter and form foam cells that is one of the initial steps in forming atherosclerotic plaques and blocking blood flow. Therefore lower expression levels of CD36 on immune cells is preventive for the formation of foam cells and progress of atherogenesis (Park Y.M. Exp Mol Med. 2014 Jun 6; 46:e99. doi: 10.1038/emm.2014.38. CD36, a scavenger receptor implicated in atherosclerosis; Nicholson AC, Trends Cardiovasc Med. 2004 Jan;14(1):8-12 Expression of CD36 in macrophages and atherosclerosis: the role of lipid regulation of PPARgamma signaling). The combination especially of upregulating ABCA1 and downregulating CD36 could be even more powerful in attenuating atherosclerosis (Rubic T, Lorenz RL, Cardiovasc Res. 2006 Feb 1;69(2):527-35. Epub 2005 Dec 5; Downregulated CD36 and oxLDL uptake and stimulated ABCA1/G1 and cholesterol efflux as anti-atherosclerotic mechanisms of interleukin-10).

Other factors that can prevent progression of atherogenesis are higher levels of IL-19 expression, which may also prevent inflammation(Gabunia K, Ellison S, Kelemen S, Kako F, Cornwell WD, Rogers TJ, Datta PK, Ouimet M, Moore KJ, Autieri MV.Am J Pathol. 2016 May;186(5):1361-74. doi:10.1016/j.ajpath.2015.12.023. Epub 2016 Mar 4; IL-19 Halts Progression of Atherosclerotic Plaque, Polarizes, and Increases Cholesterol Uptake and Efflux in Macrophages) as well as short chain fatty acids in circulation that can act via the GPR43/FFAR2 receptor (Ohira H, Tsutsui W, Fujioka Y.; J Atheroscler Thromb. 2017 Jul 1;24(7):660-672. doi: 10.5551/jat.RV17006. Epub 2017 May 27. Are Short Chain Fatty Acids in Gut Microbiota Defensive Players for Inflammation and Atherosclerosis?).

Human Milk Oligosaccharides are ingredients of human milk that can be absorbed from the intestine and have an effect on the immune system in circulation (Triantis V, Bode L, van Neerven RJJ; Front Pediatr. 2018 Jul 2;6:190. doi: 10.3389/fped.2018.00190. eCollection 2018. Immunological Effects of Human Milk Oligosaccharides).

Since human diet is containing cholesterol and fat that could contribute to obesity, atherogenesis and cardiovascular disease (from infancy with human milk to adulthood with animal products), there is a need for compounds that modulate the expression of genes relevant to cholesterol metabolism and atherogenesis.

Diabetes is a prevalent metabolic disease affecting several millions of people worldwide. Between 2019 and 2045 the global expenditures for diabetes treatment is expected to grow from 760 billion U.S. dollars to 845 billion U.S. dollars per year. Diabetes can have life-threatening cardiovascular, renal and nervous system consequences when left untreated. About 90% to 95% of diabetes patients in the USA have non-insulin dependent diabetes mellitus (NIDDM) or type II diabetes. One of the hallmarks of type II diabetes is decreased sensitivity of muscle and adipose cells to insulin. Compounds that increase the sensitivity of adipose to insulin may be useful in the treatment of diabetes and its complications. Upon insulin treatment, insulin receptor is phosphorylated which activates a signal transduction pathway leading to increased glucose uptake by glucose transporter 4 (GLUT4) in adipocytes (fat) or myocytes (muscle). Therefore, measuring glucose uptake provides the most relevant end point assay for insulin sensitivity.

It is desired to identify compounds that may modulate the gene expression for CD36, ABCA1, GPR43/FFAR2 and IL19. In particular it is desired to downregulate the expression for CD36 and /or to decrease the CD36 receptor levels. It is also desired to have food compositions comprising one or more of such compounds that can help in the prevention of atherosclerosis and / or reduce the amount of body fat for example by increasing lipolysis. Likewise, it is desired that compounds modulate the glucose uptake, in particular increase the glucose uptake. It is further desired that such compound(s) may be used in food products, in particular for adult nutrition. It is also desired that such compound(s) is/are considered safe, preferably have a GRAS status (Generally Recognized As Safe; sections 201(s) and 409 of the Federal Food, Drug, and Cosmetic, FDA).

It is an objective of the present invention to provide a composition that addresses at least one of the aforementioned desires and or needs.

SUMMARY OF THE INVENTION

It was surprisingly found that compounds according to formula 1,

$$Y_n \rightarrow D\text{-Gal}\beta1 \rightarrow 3\text{-}(Y_m \rightarrow)(X_p \rightarrow)D\text{-GlcNAc-}\beta1 \rightarrow 3\text{-D-Gal-}\beta1 \rightarrow 4\text{-}(X_q \rightarrow)\text{-D-Glc} \qquad \text{formula 1}$$

wherein

X is $\alpha$-L-Fucose ($\alpha$-L-Fuc),
Y is N-acetyl-D-neuraminic acid (Neu5Ac-$\alpha$-2-)
n is 0 or 1
m is 0 or 1
p is 0 or 1
q is 0 or 1
and

$$n+m+p+q \geq 1;$$

are able to modulate the expression of genes related to high fat diet, atherogenesis and cardiovascular disease. Compounds according to formula 1 regulate beneficially the expression of genes related to high fat diet, atherogenesis and cardiovascular disease. These compounds further are able to increase lipolysis and reduce the risk for atherosclerosis. The composition of the invention may be used in therapeutic and non-therapeutic treatments. Non-therapeutic treatments are considered cosmetic treatments such as maintaining or keeping a healthy body weight.

The invention is set out in the appended set of claims.

In particular, the synthetic composition of the invention is used in a reduction of the cholesterol level.

DETAILED DESCRIPTION OF THE INVENTION

The term "treatment", in relation a given disease or disorder, includes, but is not limited to, inhibiting the disease or disorder, for example, arresting the development of the disease or disorder; relieving the disease or disorder, for example, causing regression of the disease or disorder; or relieving a condition caused by or resulting from the disease or disorder, for example, relieving, preventing or treating symptoms of the disease or disorder.

The term "prevention" in relation to a given disease or disorder means preventing the onset of disease development if none had occurred, preventing the disease or disorder from occurring in a subject that may be predisposed to the disorder or disease but has not yet been diagnosed as having the disorder or disease, and/or preventing further disease/disorder development if already present.

It is also to be understood that this invention is not limited to the specific embodiments and methods described herein, as specific components and/or conditions may, of course, vary. Furthermore, the terminology used herein is used only for the purpose of describing particular embodiments of the present invention and is not intended to be limiting in any way.

It must also be noted that, as used in the specification and the appended claims, the singular form "a", "an," and "the" comprise plural referents unless the context clearly indicates otherwise. For example, reference to a component in the singular is intended to comprise a plurality of components.

It will be understood that within this disclosure, any reference to a weight, weight ratio, and the like pertains to the dry matter, in particular the dry matter of the composition.

Unless defined otherwise, all technical and scientific terms used herein generally have the same meaning as commonly understood by one of ordinary skill in the art to which this invention belongs.

As used herein, the term "comprising", which is synonymous with "including" or "containing", is open-ended, and does not exclude additional, unrecited element(s), ingredient(s) or method step(s), whereas the term "consisting of" is a closed term, which excludes any additional element, step, or ingredient which is not explicitly recited.

The term "subject" as used herein refers to a human, that is treatable by the method of the invention. The term "subject" refers to both the male and female sex unless one sex is specifically indicated. The human subject can be an infant, a juvenile, an adolescent, an adult or an elderly subject. The human subject can have an age of between 0 - 3 months, 0 - 6 months, 3 - 6 months, 0 - 12 months, 6 - 12 months, 12 - 24 months, 12 - 36 months, it can have an age of up to 5 years, up to 10, 12, 15, 20, or 30 years; or an age $\geq$ 30 years such as $\geq$ 40 year, $\geq$ 45 years, $\geq$50 years, $\geq$55 years, $\geq$ 60 years, $\geq$65 years, $\geq$70 years, $\geq$ 75 years, $\geq$80 years or even $\geq$85 years.

In embodiments of the invention the human subject is at least 18 years of age, e.g. at least 25 years, at least 30 years, at least 35 years, at least 40 years, at least 45 years, at least 50 years, at least 55 years, at least 60 years or at least 65 years of age. There is no particular upper limit although in practice, human subjects treated in accordance with the invention will typically be at most 100 years of age, e.g. at most 95 or at most 90 years of age.

As used herein, the term D-Gal refers to D-galactopyranose. The term D-GlcNAc refers to D-(Acetylamino)-2-deoxy-glucopyranose. L-Fuc refers to L-fucopyranose. D-Glc refers to D-glucopyranose. The "$\alpha$" or "$\beta$" directly following the monosaccharide abbreviation (e.g. Glc, Gal, Fuc) indicate the chirality of the anomeric carbon. So, D-Gal-$\beta$1$\rightarrow$4-D-Glc represents a lactose moiety i.e. a $\beta$-D-galactopyranose linked to the 4 position of D-glucose, with a $\beta$1 $\rightarrow$ 4 linkage. Likewise, Neu5Ac-$\alpha$-2$\rightarrow$ 3- D-Gal represents an N-acetyl-D-neuraminic acid (i.e. 5-acetamido-3,5-dideoxy-D-glycero-D-galacto-non-2-ulopyranosonic acid) residue linked with its anomeric carbon (i.e. carbon 2) in alpha configuration to the 3 position of a D-galactose residue (i.e. an $\alpha$-2$\rightarrow$ 3 linkage). If there is no number specified at the right-hand side of the linkage arrow, then the linkage may be to any free OH-group of the monosaccharide residue indicated, except for the anomeric OH. So if $X_q$ is defined with X is $\alpha$-L-Fucose ($\alpha$-L-Fuc) and q equals 1, then D-Gal-$\beta$1$\rightarrow$4-($X_q\rightarrow$)D-Glc represents a lactose moiety (D-Gal-$\beta$1$\rightarrow$4-D-Glc) wherein an $\alpha$-L-fucose residue is linked to the 2, 3, or 6 position of the glucose residue (an L-Fuc-$\alpha$1$\rightarrow$2 linkage or L-Fuc-$\alpha$1$\rightarrow$3 linkage or L-Fuc-$\alpha$1$\rightarrow$6 linkage, respectively).

As used herein, $(Y_m\rightarrow)(X_p\rightarrow)$D-GlcNAc means that both substituent Y and X may be linked to the D-GlcNAc residue, depending on the definition of m and p. It is further understood that Y and X cannot be linked to the same carbon atom at the same time.

"Effective amount" or "therapeutically effective amount" as used herein, refers to an amount of compound of formula (I), or a composition thereof that is effective in producing the desired therapeutic, ameliorative, inhibitory, non-therapeutic or preventative effect when administered to a patient suffering from a condition. An effective amount can refer to each individual agent alone or to the combination as a whole, wherein the amounts of all agents administered are together effective, but wherein the component agent of the combination may not be present individually in an effective amount.

"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. Preferably, the solvate is a hydrate. "Hydrate" is a solvate wherein the solvent molecule is $H_2O$.

The compounds of formula 1 especially those comprising an acidic moiety, can form salts which are also within the scope of this invention. Reference to a compound of formula 1 herein is understood to include reference to salts thereof, unless otherwise indicated. The term "salt(s)", as employed herein, denotes salts formed with organic acids (compound of formula 1) and organic or inorganic bases. Pharmaceutically acceptable (i.e., non-toxic, physiologically acceptable) salts are preferred, although other salts are also useful. Salts of the compounds of the formula 1 may be formed, for example, by reacting a compound of formula 1 with an amount of base, such as an equivalent amount, in a medium such as one in which the salt precipitates or in an aqueous medium followed by lyophilization.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, lysine and the like. All such salts are intended to be pharmaceutically acceptable salts within the scope of the invention and all salts are considered equivalent to the free forms of the corresponding compounds for purposes of the invention. The salts are intended to be acceptable for the subject using the composition of the invention. So, if the composition is an infant formula, then the salt is a salt acceptable in infant formula.

Pharmaceutically acceptable esters of the present compounds include carboxylic acid esters obtained by esterification of the carboxyl group of formula 1 with an alcohol (for example, methanol, ethanol, n-propanol). Preferably, the acid group of the compound of formula 1 is not esterified.

The term "infant," as used herein, unless otherwise specified, refers to a human 36 months of age or younger. The term "toddler," as used herein, unless otherwise specified, refers to a subgroup of infant that is 12 months of age to 36 months of age. The term "child," as used herein, unless otherwise specified, refers to a human 3 years of age to 18 years of age. The term "adult, " as used herein, unless otherwise specified, refers to a human 18 years of age or older.

The term "synthetic composition" as used herein refers to a composition which is artificially prepared and preferably means a composition comprising at least one compound that is produced *ex vivo* chemically and/or biologically, e.g. by means of chemical reaction, enzymatic reaction or recombinantly, or purified by humans. Preferably, the synthetic composition of the invention is not identical with a naturally occurring composition. The synthetic composition of the invention typically comprises one or more compounds, advantageously HMOs, but may further include other ingredients like protein, fat, minerals or vitamins.

As used herein an adult person is said to have a "healthy body weight" if the person has a Body mass index (BMI) between 18.5 and 25; wherein the BMI is defined as the body mass divided by the square of the body height, and is universally expressed in units of kg/m2, resulting from mass in kilograms and height in meters.

For children, age 18 or below, the BMI is compared against the percentiles for children of the same sex and age. A BMI that is less than the 5th percentile is considered underweight and above the 95th percentile is considered obese. Children with a BMI between the 85th and 95th percentile are considered to be overweight. Children with a BMI between the 5th and the 85th percentile are considered to have a healthy weight.

So, in a first aspect the invention relates to a synthetic composition for use as defined in claim 1.

The use of the synthetic composition of the invention in a non-therapeutic use is disclosed. As used herein, a non-therapeutic use is considered a cosmetic use.

The compound according to formula 1 is a Human Milk Oligosaccharide (HMO) and may be obtained from commercial suppliers, it may be synthesized using conventional organic chemistry methods, or it may be isolated from milk, e.g human milk using methods known in the art. Briefly, isolation from (human) milk may be done by obtaining (human) milk from volunteers of (preterm) infants. After centrifugation of the milk, the lipid layer is removed and proteins precipitated from the aqueous phase by addition of ice-cold ethanol and subsequent centrifugation. Ethanol can be removed from the HMO- containing supernatant by roto-evaporation. Oligosaccharides may then be separated using anion exchange chromatography, particularly high-pH anion exchange chromatography with pulsed amperometric detection, Prior separation of the neutral and acidic oligosaccharides may be required. Alternatively, HMO may be separated using reverse phase (RP) HPLC (Luhaak et al, Advances in Analysis of Human Milk Oligosaccharides Article in American Society for Nutrition, Advances in Nutrition 3, 4065 - 4145, May 2012; DOI: 10.3945/an.112.001883 Source: PubMed).

In one embodiment, the invention relates to the synthetic composition of the invention for use in the reduction of the cholesterol level.

In still another embodiment, the synthetic composition of the invention comprises two or more compounds according to formula 1.

Preferably, the compound according to formula 1 is selected from the group consisting of lactodifucoheaxose II (LNDFHII, CAS# 62258-12-2), sialyllacto-N-tetraose a (LSTa, CAS# 64003-58-5) and disialyllacto-N-tetraose (DSLNT, CAS# 61278-38-4), or a pharmaceutically accepted salt, ester or solvate thereof. Preferably, the solvate is a hydrate. More preferably, the synthetic composition for use according to the present invention comprises two compounds of formula 1, both selected from the group consisting of lactodifucoheaxose II (LNDFHII), sialyllacto-N-tetraose a (LSTa) and disialyllacto-N-tetraose (DSLNT). Even more preferably, the composition comprises lactodifucoheaxose II (LNDFHII), sialyllacto-N-tetraose a (LSTa) and disialyllacto-N-tetraose (DSLNT).

The amount of compound according to formula 1 in the composition of the invention preferably is between 0.001 wt% and 15 wt% of the dry weight of the composition, preferably between 0.001 and 10 wt%, more preferably between 0.01 and 5 wt%. Particularly preferably, the total amount of oligosaccharides with a degree of polymerisation of from 3 to and including 10 in the composition of the invention is between 0.001 wt% and 30 wt%, preferably below 25 wt% of the dry weight of the composition, more preferably between 0.01 and 20 wt%, even more preferably between 0.1 and 15 wt% of the dry weight of the composition.

In one embodiment, the amount of compound according to formula 1 in the composition of the invention preferably is between 0.001 gram and 15 gram per serving, preferably between 0.001 and 10 gram, more preferably between 0.01 and 5 gram. Particularly preferably, the total amount of oligosaccharides excluding lactose and excluding the optional non-digestible oligosaccharides (e.g. GOS, or FOS), in the composition of the invention is between 0.001 gram and 25 gram per serving, preferably between 0.001 and 20 gram, more preferably between 0.005 and 20 gram even more preferably between 0.01 and 10 gram per serving.

The synthetic composition of the invention may further comprise at least one source of fat, at least one source of protein and at least one source of carbohydrate. Generally, any source of protein, carbohydrate, or fat that is suitable for use in nutritional products is also suitable for use herein, provided that such macronutrients are also compatible with the essential elements of the nutritional composition as defined herein.

In one embodiment, the synthetic composition of the invention is an infant formula, preferably, wherein the composition is a formula for children having an age selected from the group consisting of 0-6 months, 0-12 months, 6-12 months 12-24 months, 12-36 months and 24-36 months. More preferably, wherein the composition is a formula for children having an age of 0-6 months, 0-12 months, or 12-36 months. In another embodiment the composition is an adult formula.

The terms "infant formula" or "infant nutritional product" as used herein are used interchangeably to refer to nutritional compositions that have the proper balance of macronutrients, micro-nutrients, and calories to provide sole or supplemental nourishment for and generally maintain or improve the health of infants, toddlers, or both. Infant formulas preferably comprise nutrients in accordance with the relevant infant formula guidelines for the targeted consumer or user population, an example of which would be the Infant Formula Act, 21 U.S.C. Section 350(a). Another example with guidelines for nutrients of an infant formula, in particular for a person of 0-12 months of age and for children up to 36 months old, may be found in the CODEX Alimentarius (CODEX STAN 72-1981), further referred to as the CODEX). Nutritional compositions for infants are commonly referred to as infant formula. When used as infant formula, the composition as used in the various aspects of the invention should contain the ingredients in the amounts as prescribed by the CODEX and, if needed, as prescribed by additional regulations of individual countries. An example of an ingredient list of an infant formula meeting the requirements of the EU, China and Codex can for example be found on www.frieslandcampinaingredients.com/ at app/uploads/2019/04/PDS_ELN_Essential®-Start-IF-110.pdf.

In certain embodiments, when the nutritional powder is formulated as an infant formula, the protein component is typically present in an amount of from 5% to 35% by weight of the infant formula (i.e., the dry weight), including from 10% to 30%, from 10% to 25%, from 15% to 25%, from 20% to 30%, from 15% to 20%, and also including from 10% to 16% by weight of the infant formula (i.e., the dry weight). The carbohydrate component is typically present in an amount of from 40% to 75% by weight of the infant formula (i.e., the dry weight), including from 45% to 75%, from 45% to 70%, from 50% to 70%, from 50% to 65%, from 50% to 60%, from 60% to 75%, from 55% to 65%, and also including from 65% to 70% by weight of the infant formula (i.e., the dry weight). The fat component is typically present in an amount of from 10% to 40% by weight of the infant formula (i.e., the dry weight), including from 15% to 40%, from 20% to 35%, from 20% to 30%, from 25% to 35%, and also including from 25% to 30% by weight of the infant formula (i.e., the dry weight).

In certain embodiments, when the nutritional powder is formulated as a pediatric formula, the protein component is typically present in an amount of from 5% to 30% by weight of the pediatric formula (i.e., the dry weight). The carbohydrate component is typically present in an amount of from 40% to 75% by weight of the pediatric formula, (i.e., the dry weight). The fat component is typically present in an amount of from 10% to 25% by weight of the pediatric formula, (i.e., the dry weight).

Alternatively, the composition of the invention is a product aimed at adults, i.e. an adult formula. As used herein, the terms "adult formula" and "adult nutritional product" as used herein are used interchangeably to refer to nutritional compositions suitable for generally maintaining or improving the health of an adult. When the nutritional powder is formulated as an adult nutritional product, the protein component is typically present in an amount of from 5% to 35% by weight of the adult nutritional product, including from 10% to 25%, and including from 20% to 30% by weight of the adult nutritional product (dry weight). The carbohydrate component is typically present in an amount of from 40% to 80% by weight of the adult nutritional product, including from 50% to 75%, and also including from 60% to 75% by weight of the adult nutritional product. The fat component is typically present in an amount of from 0.5% to 20%, including from 1% to 15%, and also including from 15% to 20% by weight of the adult nutritional product.

Generally, any source of protein may be used so long as it is suitable for oral nutritional compositions and is otherwise compatible with any other selected ingredients or features in the nutritional composition. Non-limiting examples of suitable proteins (and sources thereof) suitable for use in the nutritional powders described herein include, but are not limited to, intact, hydrolyzed, or partially hydrolyzed protein, which may be derived from any known or otherwise suitable source such as milk (e.g., casein, whey), animal (e.g., meat, fish), cereal (e.g., rice, corn, wheat), vegetable (e.g., soy, pea, potato, bean), and combinations thereof. The protein may also include a mixture of amino acids (often described as free amino acids) known for use in nutritional products or a combination of such amino acids with the intact, hydrolyzed, or partially hydrolyzed proteins described herein. The amino acids may be naturally occurring or synthetic amino acids.

More particular examples of suitable protein (or sources thereof) used in the nutritional powders disclosed herein include, but are not limited to, whole cow's milk, partially or completely defatted milk, milk protein concentrates, milk protein isolates, nonfat dry milk, condensed skim milk, whey protein concentrates, whey protein isolates, acid caseins, sodium caseinates, calcium caseinates, potassium caseinates, legume protein, soy protein concentrates, soy protein isolates, pea protein concentrates, pea protein isolates, collagen proteins, potato proteins, rice proteins, wheat proteins, canola proteins, quinoa, insect proteins, earthworm proteins, fungal (e.g., mushroom) proteins, hydrolyzed yeast, gelatin, bovine colostrum, human colostrum, glycol macropeptides, mycoproteins, proteins expressed by microorganisms (e.g., bacteria and algae), and combinations thereof. The nutritional powders described herein may include any individual source of protein or combination of the various sources of protein listed above. In addition, the proteins for use herein can also include, or be entirely or partially replaced by, free amino acids known for use in nutritional products, non-limiting examples of which include L-tryptophan, L-glutamine, L-tyrosine, L-methionine, L-cysteine, taurine, L-arginine, L-carnitine, and combinations thereof.

The carbohydrate or source of carbohydrate suitable for use in the composition disclosed herein may be simple, complex, or variations or combinations thereof. Generally, the carbohydrate may include any carbohydrate or carbohydrate source that is suitable for use in oral nutritional compositions and is otherwise compatible with any other selected ingredients or features in the nutritional powder. Non-limiting examples of carbohydrates suitable for use in the nutritional powders described herein include, but are not limited to, polydextrose, maltodextrin; hydrolyzed or modified starch or cornstarch; glucose polymers; corn syrup; corn syrup solids; sucrose; glucose; fructose; lactose; high fructose corn syrup; honey; sugar alcohols (e.g., maltitol, erythritol, sorbitol); isomaltulose; sucromalt; pullulan; potato starch; and other slowly-digested carbohydrates; dietary fibers including, but not limited to, fructooligosaccharides (FOS), galactooligosaccharides (GOS), oat fiber, soy fiber, gum arabic, sodium carboxymethylcellulose, methylcellulose, guar gum, gellan gum, locust bean gum, konjac flour, hydroxypropyl methylcellulose, tragacanth gum, karaya gum, gum acacia, chitosan, arabinogalactans, glucomannan, xanthan gum, alginate, pectin, low methoxy pectin, high methoxy pectin, cereal beta-glucans (e.g., oat beta-glucan, barley beta-glucan), carrageenan and psyllium, soluble and insoluble fibers derived from fruits or vegetables; other resistant starches; and combinations thereof. The nutritional powders described herein may include any individual source of carbohydrate or combination of the various sources of carbohydrate listed above.

The fat or source of fat suitable for use in the nutritional powders described herein may be derived from various sources including, but not limited to, plants, animals, and combinations thereof. Generally, the fat may include any fat or fat source that is suitable for use in oral nutritional compositions and is otherwise compatible with any other selected ingredients or features in the nutritional powder. Non-limiting examples of suitable fat (or sources thereof) for use in the nutritional powders disclosed herein include coconut oil, fractionated coconut oil, soy oil, high oleic soy oil, corn oil, olive oil, safflower oil, high oleic safflower oil, medium chain triglyceride oil (MCT oil), high gamma linolenic (GLA) safflower oil, sunflower oil, high oleic sunflower oil, palm oil, palm kernel oil, palm olein, canola oil, high oleic canola oil, marine oils, fish oils, algal oils, borage oil, cottonseed oil, fungal oils, eicosapentaenoic acid (EPA), docosahexaenoic acid (DHA), arachidonic acid (ARA), conjugated linoleic acid (CLA), alpha-linolenic acid, rice bran oil, wheat bran oil, interesterified oils, transesterified oils, structured lipids, and combinations thereof. Generally, the fats used in nutritional powders for formulating infant formulas and pediatric formulas provide fatty acids needed both as an energy source and for the healthy development of the infant, toddler, or child. These fats typically comprise triglycerides, although the fats may also comprise diglycerides, monoglycerides, and free fatty acids. Fatty acids provided by the fats in the nutritional powder include, but are not limited to, capric acid, lauric acid, myristic acid, palmitic acid, palmitoleic acid, stearic acid, oleic acid, linoleic acid, alpha-linolenic acid, ARA, EPA, and DHA. The nutritional powders can include any individual source of fat or combination of the various sources of fat listed above. Preferably, the fat is a mixture of vegetable fat and milk fat such as obtained from milk from a mammal like cow, sheep, goat, mare, or camel. More preferably, wherein the milk fat is bovine milk fat. Mixtures of different types of fat are preferred because they help to provide different fatty acids and better resemble the type of linkage between the glycerol moiety and the fatty acid moiety in the fat, when compared to human mother's milk.

In certain embodiments, the nutritional powders described herein may further comprise other optional ingredients that may modify the physical, chemical, hedonic, or processing characteristics of the products or serve as additional nutritional components when used for a targeted population. Many such optional ingredients are known or otherwise suitable for use in other nutritional products and may also be used in the nutritional powders described herein, provided that such optional ingredients are safe and effective for oral administration and are compatible with the essential and other ingredients in the selected product form. Non-limiting examples of such optional ingredients include preservatives, antioxidants, emulsifying agents, buffers, additional nutrients as described herein, colorants, flavors (natural, artificial, or both), thickening agents, flow agents, anti-caking agents, and stabilizers.

In certain embodiments, the nutritional powders further comprise minerals, non-limiting examples of which include calcium, phosphorus, magnesium, iron, zinc, manganese, copper, sodium, potassium, molybdenum, chromium, selenium, chloride, and combinations thereof.

In certain embodiments, the nutritional powders further comprise vitamins or related nutrients, non-limiting examples of which include vitamin A, vitamin D, vitamin E, vitamin K, thiamine, riboflavin, pyridoxine, vitamin B12, niacin, folic acid, pantothenic acid, biotin, vitamin C, choline, inositol, salts and derivatives thereof, and combinations thereof.

The composition of the invention may be powder (or powder product), a pod or a liquid (or liquid product). The term "pod" as used herein, unless otherwise specified, refers to a sealable, re-sealable or sealed container having an internal volume capable of containing a solid, powder, or liquid formulation that, when mixed with liquid, yields a liquid product suitable for human consumption.

The term "liquid product" as used herein, unless otherwise specified, refers to the reconstituted nutritional powder.

The term "nutritional powder" as used herein, unless otherwise specified, refers to nutritional products that are solids or semisolids in the form of particles that are generally flowable or scoopable. A nutritional powder is usually reconstituted by addition of water or another liquid to form a liquid nutritional composition prior to administration to (e.g., providing to or consumption by) an individual. As discussed below, in certain embodiments disclosed herein, the nutritional powders comprise at least one of a source of protein, a source of carbohydrate, and a source of fat.

The terms "reconstitute," "reconstituted," and "reconstitution" as used herein, unless otherwise specified, are used to refer to a process by which the nutritional powder is mixed with a liquid, such as water, to form an essentially homogeneous liquid product. Once reconstituted in the liquid, the ingredients of the nutritional powder may be any combination of dissolved, dispersed, suspended, colloidally suspended, emulsified, or otherwise blended within the liquid matrix of the liquid product. Therefore, the resulting reconstituted liquid product may be characterized as any combination of a solution, a dispersion, a suspension, a colloidal suspension, an emulsion, or a homogeneous blend.

The term "serving" as used herein, unless otherwise specified, is any amount of a composition that is intended to be ingested by a subject in one sitting or within less than about one hour. The size of a serving (i.e., "serving size") may be different for diverse individuals, depending on one or more factors including, but not limited to, age, body mass, gender, species, or health. For a typical human child or adult, a serving size of the compositions disclosed herein is from about 25 mL to 1,000 mL, or when taken as a solid product from 30 g to 250 g, such as from 100 to 15 g. For a typical human infant or toddler, a serving size of the compositions disclosed herein is from about 5 mL to about 250 mL.

In one embodiment the synthetic composition is a food product selected from the group consisting of confectionary products; nutritionally complete food products; desserts, in particular a pudding, yoghurt, custard, vla, ice-cream, or milk-shake; beverages, in particular a fruit juice or milk; breakfasts, such as porridge, cereals; soups; and sauces. A preferred food product is a confectionary product selected from the group of food-bar (such as granola bars, candy bars), sweeties and cookies.

The composition of the invention may further comprise human milk oligosaccharides (HMO, or HMOs) other than a compound according to formula 1. HMOs are oligosaccharides that occur in human milk. Human milk oligosaccharides (HMOs) are a key constituent of human milk. They are a structurally and biologically diverse group of complex indigestible carbohydrates. To date, more than 200 different oligosaccharides have been identified, varying in size from 3 to 22 monosaccharide units. The most common HMOs are the neutral fucosylated and non-fucosylated oligosaccharides. The quantity and structure of these HMOs differ significantly among women and is dependent upon Secretor and Lewis blood group status (L. Bode, J. Nutr. 136: 2127-2130, 2006.). In one embodiment, the composition as used in the aspects of the invention comprises one or more HMOs.

The HMOs of human milk are composed of various monosaccharides, namely glucose, galactose, fucose, N-acetylglucosamine and sialic acids (N-acetylneuraminic acid). The sugar fucose is an unusual molecule in that it has the L-configuration, whereas the other sugar molecules in the body have the D-configuration. The structure of HMOs is a lactose unit which may be elongated with one or more galactose and / or N-acetylglucosamine residues (core structure). The HMO core structure may be decorated with one or more fucose residues (i.e. fucosylated HMO) and with one or more sialic acid units (i.e. sialylated HMO). A HMO may also be fucosylated and sialylated. In one embodiment, the HMO in the composition of the invention is selected from one or more the group consisting of core HMO, sialylated HMO, and fucosylated HMO. Nearly 200 HMOs have been identified from human milk. Fucosylated HMOs were found to be the most prominent component (~77%), while sialylated HMOs accounted for about 16% of the total abundance of HMOs. The fucosylated HMOs are neutral molecules, while the sialylated HMOs are acidic. In human milk, the most abundant HMO is 2'-fucosyllactose (a neutral trisaccharide composed of L-fucose, D-galactose, and D-glucose units, linked Fuc($\alpha$1-2) Gal($\beta$1-4)Glc; CAS Nr 41263-94-9), with a concentration of about 2 g/l (Adams et al; 2018, Nutrafoods pp 169 - 173). Preferred HMOs are 3'-Sialyllactose (3'SL); 6'-Sialyllactose (6'SL); 2'- Fucosyllactose (2'FL); 3-Fucosyllactose (3-FL); lacto-N-tetraose (LNT), lacto-N-neotetraose (LNnT), LNFP II, LNFP III, and disialyllacto-N-tetraose (DSLNT). Particularly preferred nutritional compositions include at least 2'FL. HMOs can be obtained using methods known to those of skill in the art. For example, HMOs can be purified from human milk. Individual HMOs can be further separated using methods known in the art such as capillary electrophoresis, HPLC (e.g., high-performance anion-exchange chromatography with pulsed amperometric detection; HPAEC-PAD), and thin layer chromatography. See, e.g., U.S. Patent Application No.

2009/0098240. Alternately, enzymatic methods can be used to synthesize HMOs. Another method to manufacture HMO's is via biosynthesis in engineered bacteria. For example, a method of preparing 2'-FL is disclosed in WO 2012/112777. Alternatively, 2'-FL is commercially available e.g. from FrieslandCampina, or others.

The inventors have surprisingly found that when the composition of the invention further comprises LNFP II or LNFP III, more preferably when it further comprises both LNFP II and LNFP III it is more effective in increasing the glucose uptake. So, in one embodiment the composition of the invention further comprises LNFP II or LNFP III, more preferably, the composition further comprises LNFP II and LNFP III.

As used herein, LNFP II refers to Lacto-N-fucopentaose-2, i.e. β-D-Gal-(1→3)-(α-L-Fuc-[1→4])-β-D-GlcNAc-(1→3)-β-D-Gal-(1→4)-D-Glc, i.e. D-Glucose, O-6-deoxy-alpha-L-galactopyranosyl-(1-4)-O-(beta-D-galactopyranosyl-(1-3))-O-2-(acetylamino)-2-deoxy-beta-D-glucopyranosyl-(1-3)-O-beta-D-galactopyranosyl-(1-4)-;      CAS number 21973-23-9.

As used herein LNFP III refers to Lacto-N-fucopentaose III, i.e. β-D-Gal-(1→4)-[α-L-Fuc-(1→3)]-β-D-GlcNAc-(1→3)-β-D-Gal-(1→4)-D-Glc; CAS number 25541-09-7.

The composition of the invention may further comprise one or more probiotics and / or one or more prebiotics. Probiotics and prebiotics are known in the art and are claimed to have beneficial effect on the subject's gut microbiome and to have a positive effect on the subject's health and / or wellbeing.

Non-digestible oligosaccharides are a class of prebiotics. In another embodiment of the invention, the composition comprises 0.25 to 20 wt.% non-digestible oligosaccharides based on dry weight of the composition, preferably wherein the non-digestible oligosaccharides are selected from one or more of galacto-oligosaccharides (GOS), and fructo-oligosaccharides (FOS), more preferably, wherein the non-digestible oligosaccharides are galacto-oligosaccharides. In other embodiments the minimum amount of non-digestible oligosaccharides is at least 1 wt% based on dry weight of the composition, such as at least 5 wt%. In yet another embodiment, the maximum amount of non-digestible oligosaccharide is 25 wt% based on dry weight of the composition, preferably less than 20 wt%, more preferably less than 15 wt%. Preferably, the composition comprises between 0.25 and 20 wt% GOS, more preferably between 1 and 10 wt% GOS, based on dry weight of the composition. GOS and FOS are commercially available and FOS may include inulin.

Probiotics are live microorganisms promoted with claims that they provide health benefits when consumed, generally by improving or restoring the gut flora. Probiotics are well known in the art and examples include Saccharomyces boulardii (a yeast) and bacteria in the Lactobacillus and Bifobacterium families of microorganisms. Lactobacillus acidophilus is the probiotic that is found in some yogurts.

Adiponectin (also referred to as GBP-28, apM1, AdipoQ and Acrp30) has repeatedly been shown to be negatively associated with bone mass in humans.

So, preferably, the composition used in the various aspects and embodiments of the invention comprises less than 0.1 wt% of adiponectin or biologically active fragments thereof, more preferably less than 0.01 wt%, even more preferably less than 0.001 wt% of adiponectin, as determined to the dry weight of the composition. Most preferably, the composition does not comprise adiponectin or biologically active fragments thereof.

Lactoferrin (LF) is a multifunctional glycoprotein found in mammalian breast milk. Its primary function was thought to be antimicrobial or immunomodulatory activity because it is present at high concentrations in breast milk. In recent years, several studies have shown other functions of LF, including modulation of lipid metabolism. *In vitro* studies have revealed anti-adipogenic and lipolytic activities of LF against adipocytes. *In vivo* studies have also demonstrated that LF can decrease plasma, hepatic triglycerides and cholesterol and can reduce visceral fat. So in yet another embodiment, the composition as used in the various aspects and embodiments of the invention further comprises lactoferrin. Preferably the amount of LF is more than 0.01 wt% of dry composition, more preferably more than 0.1 wt%. Normally the amount of LF does not exceed 5 wt%, preferably it is less than 3 wt%, more preferably less than 1 wt%. In another embodiment the amount of LF is more than 0.1 g per liter of composition.

Soluble Cluster of Differentiation 14 (sCD14) is a protein present at low concentrations in human milk and bovine milk. sCD14 mediates secretion of innate immune response molecules such as interleukin-8, tumor necrosis factor-alpha, and epithelial neutrophil activator-78 by CD14-negative intestinal epithelial cells exposed to lipopolysaccharide (LPS) or bacteria. The inventors surprisingly found that the combination of sCD14 with the compounds of formula 1 as present in the synthetic composition of the invention further decrease the production of CD36 as compared to the administration of the compounds of formula 1. So in yet another embodiment, the synthetic composition of the invention comprising one or more compounds of formula 1, further comprises sCD14, preferably, it comprises sCD14 in a therapeutically effective amount, more preferably it comprises at least 0.0001 wt% of sCD14, more preferably at least 0.001. The amount of sCD14 normally does not exceed 0.01 wt%, preferably it is less than 0.005 wt%, as determined to the dry weight of the composition. So, in one embodiment the composition further comprises between 0.0001 and 0.01 wt% of sCD14.

The synthetic composition comprising the compound(s) of formula 1 may also be used for use in medicine, for the indications cited herein. Preferably for use in a human subject.

Except in the examples, or where otherwise expressly indicated, all numerical quantities in this description indicating amounts of material or conditions of reaction and/or use are to be understood as modified by the word "about" in describing the broadest scope of the invention. Practice within the numerical limits stated is generally preferred. Also, unless expressly stated to the contrary: percent, "parts of," and ratio values are by weight; the description of a group or class of materials as suitable or preferred for a given purpose in connection with the invention implies that mixtures of any two or more of the members of the group or class are equally suitable or preferred; description of constituents in chemical terms refers to the constituents at the time of addition to any combination specified in the description, and does not necessarily preclude chemical interactions among the constituents of a mixture once mixed; the first definition of an acronym or other abbreviation applies to all subsequent uses herein of the same abbreviation and applies, *mutatis mutandis,* to normal grammatical variations of the initially defined abbreviation; and, unless expressly stated to the contrary, measurement of a property is determined by the same technique as previously or later referenced for the same property.

The invention is hereinafter illustrated with reference to the following, non-limiting, examples.

EXAMPLES

**Example** 1 PCR Data - HMO and gene expression

The effect of single HMOs on the expression of genes relevant to cholesterol metabolism and atherogenesis was tested using Peripheral Blood Mononuclear Cells (PBMCs) from 3 donors and incubated those cells with 0.5 g/l of single HMOs. The level of expression was determined using PCR.

Peripheral blood mononuclear cells (PBMC's) were isolated from a buffy coat using Sepmate tubes. The cells were diluted to a concentration of $1*10^7$ cells per ml. The PBMC's were seeded in 6 wells plates and treated with 0.5 g/l of each HMO in PBMC culturing medium for 5h prior to RNA isolation. Each HMO was mixed with 100ug/ml final concentration of Polymyxin B to ensure neutralization of possible endotoxin contamination of HMOs, while equal amounts of Polymyxin B were used as a control.

500 ng RNA, isolated from PBMCs, was translated into cDNA, using the quantitect reverse transcription kit (Qiagen). Primersets for the regulated genes (shown in the table below), were ordered at Qiagen (Quantitect primer assays). From each condition (PBMCs of 3 donors in control situation and after exposure towards HMOs (5 hrs)) cDNA (100x, 10x for genes which were low expressed) was amplified using the different primersets.

GAPDH (Glyceraldehyde-3-Phosphate Dehydrogenase) was used as a reference gene to quantify changes of gene expression between the control and the HMO-treated cells.

Primer sets are shown below

| | |
|---|---|
| GAPDH : | Hs_GAPDH_vb.1_SG QuantiTect Primer Assay |
| IL19 : | Hs_IL19_1_SG QuantiTect Primer Assay |
| CD36 : | Hs_CD36_3_SG QuantiTect Primer Assay |
| GPR43/FFAR2 : | Hs_FFAR2_2_SG QuantiTect Primer Assay |
| ABCA1 : | Hs_ABCA1_1_SG QuantiTect Primer Assay |

Results

19 commercially available HMO's that are present in human milk were tested. All gene expression values were calibrated to the Polymyxin B control of each respective donor (set arbitrary as 1). Average values for each gene compared to the Polymyxin B control are shown below (values above 1,5 fold or below 0,5 fold were considered physiologically relevant).

Thirteen HMOs (12 + 2'FL) of these did not show statistically significant changes in the expression of CD36, FFAR2 and IL19. These thirteen HMOs are the first 13 HMOs shown in the table below:

| 0.5mg/ml | CD36 | FFAR2 | IL19 | ABCA1 |
|---|---|---|---|---|
| LNFP-I | 0,85 | 0,86 | 1,89 | N/A |
| LNT | 0,93 | 0,93 | 0,93 | N/A |
| LNDFH-I | 0,86 | 1,74 | 1,20 | N/A |
| 3FL | 0,98 | 1,18 | 1,06 | N/A |
| 6'SL | 0,93 | 1,83 | 1,05 | N/A |
| LDFT | 1,00 | 1,64 | 0,75 | N/A |
| 2',3'-DF-LNH | 0,84 | 1,57 | 1,35 | N/A |

(continued)

| 0.5mg/ml | CD36 | FFAR2 | IL19 | ABCA1 |
|----------|------|-------|------|-------|
| 3S3FL | 0,90 | 1,03 | 0,94 | N/A |
| 3'SL | 0,87 | 1,25 | 1,24 | N/A |
| LSTc | 0,90 | 1,02 | 1,25 | N/A |
| LNnT | 0,93 | 0,93 | 0,93 | N/A |
| SLNFP-II | 0,87 | 1,41 | 0,97 | N/A |
| 2'FL | 0,67 | 0,72 | 1.41 | 1.09 |
| DSLNT | 0,31 | 5,92 | 1,71 | 2,02 |
| LSTa | 0,16 | 7,91 | 4,09 | 2,93 |
| LNFP-III | 0,08 | 7,33 | 18,45 | 3,54 |
| LNDFH-II | 0,16 | 3,81 | 2,74 | 2,16 |
| LNFP-II | 0,05 | 5,47 | 18,69 | 4,97 |
| LSTb | 0,75 | 7,75 | 1,31 | 2,35 |
| N/A : Not measured. | | | | |

Six HMOs that showed physiologically significant changes in the gene expression of CD36, FFAR2 and IL19 were additionally also tested in the expression of ABCA1. 2'FL was included too as it was expected that the most abundant HMO in human milk would show changes in any of these systems. The seven HMOs that were tested for the expression of ABCA1, CD36, FFAR2 and IL19 were [disialyllacto-N-tetraose (DSLNT, CAS# 61278-38-4), sialyllacto-N-tetraose a (LSTa, CAS# 64003-58-5), Lacto-N-fucopentaose III (LNFP-III, CAS# 25541-09-7), lactodifucoheaxose II (LNDFHII also referred to as LNDFH-II, CAS# 62258-12-2), Lacto-N-fucopentaose-2 (LNFP-II), sialyllacto-N-tetraose b (LSTb, CAS# 64003-54-9) and 2'-fucosyllactose (2'FL, CAS# 41263-94-9)). The expression relative to Polymyxin B control of these seven HMOs is shown in the lower part of the table above.

It was surprisingly found that 2'FL - the most abundant HMO in human milk - was not effective in upregulating or downregulating any of the genes tested, except for a minor, insignificant, increase in IL19 expression levels.

DSLNT, LSTa, LNFP-III, LNDFH-II, LNFP-II, and LSTb were shown to regulate the expression of one or more genes related to atherogenesis and cholesterol metabolism in a beneficial and protective way. All of these six HMOs tested were able to upregulate FFAR2 and hence defensive against inflammation and atherosclerosis without the need for other HMO's being present.

DSLNT, LSTa, LNFP-III, LNDFH-II, and LNFP-II were shown to upregulate the expression of IL19, which also provides protection against inflammation and atherogenesis, without the need for other HMO's being present. In particular, LSTa, LNFP-III and LNFP-II were able to upregulate IL-19. Especially LNFP-III and LNFP-II were able to upregulate IL-19 expression levels.

All HMOs tested (except 2'FL) were able to upregulate ABCA1, in particular LSTa, LNFP-III, LNFP-II and LSTb which is helpful in increasing HDL levels and is protective for atherogenesis.

All HMOs tested (except 2'FL) showed a reduced expression of CD36 which is preventive for the formation of foam cells and progress of atherogenesis. In particular DSLNT, LSTa, LNF-III, LNDFH-II and LNFP-II were able to reduce CD36.

**Example 2 Cholesterol uptake**

$1*10^7$ PBMC's were seeded in 6 well plates and incubated with a mixture of HMO's isolated from pooled human milk (obtained from University of California, San Diego, UCSD), pHMOs (0.5 mg/ml); 2'-FL (0.5 mg/ml) or no glycan at all i.e. control for 4 hrs followed by addition of 10 ug/ml Dil oxLDL (ThermoFisher Cat number L34358) for 1 more hour.

The pHMOs comprises among others DSLNT, LSTa, LNFP-III, LNDFH-II, and LNFP-II.

Cholesterol uptake was assessed by Fluorescence Activated Cell Sorting (FACS) gated on the CD14 / CD36 double positive cells (CD14+ CD36+). Results are plotted as percentages of Mean Fluorescence Intensity (MFI) values of the FACS plots compared to the no glycan control that was incubated with Dil oxLDL (column labelled "cholesterol").

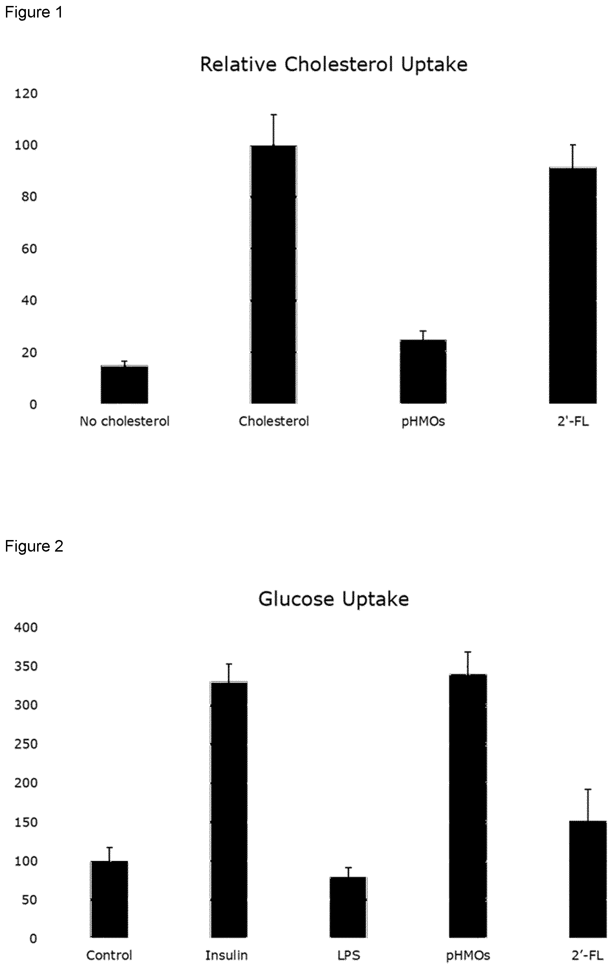

The results are shown in Figure 1. The results show that the pool of HMO's is very effective in reducing the amount of cholesterol in the cell to about 25% of the original value, while 2'FL alone surprisingly accounted for a much lower reduction of cholesterol level in the cell, as it reduced the cholesterol level to about 91 % of the original value. These results show that cells that did not receive fluorescent oxLDL (Dil-oxLDL) show some background fluorescence. When Dil-oxLDL was added in the medium then cells take it up and show lots of fluorescence (this value was set at 100%). Cells pre-incubated with pHMOs followed by Dil-oxLDL showed no take up of Dil-oxLDL and fluorescence dropped to background levels. Cells pre-incubated with 2'FL did take up Dil-oxLDL more or less in the same amounts as cells that had seen no HMO. Therefore indeed uptake of cholesterol is reduced by administering pHMOs.

**Example 3 Glucose uptake** (reference example) **Culture media (all obtained from Zen-Bio Inc):**

**Pre Adipocyte growth media (PM-1):**

| DMEM/ Ham's F-12 (1:1, v/v) | HEPES buffer |
|---|---|
| Fetal bovine serum | Antibiotics |

**Adipocyte differentiation media (DM-2):**

| DMEM/ Ham's F-12 (1:1, v/v) | HEPES buffer |
|---|---|
| Fetal bovine serum | Biotin |
| Pantothenate | Human insulin |
| Dexamethasone | 3-isobutyl-1-methylxanthine (IBMX) |
| $PPAR_Y$ agonist | Antibiotics |

**Adipocyte maintenance media (AM-2):**

| DMEM/ Ham's F-12 | HEPES buffer |
|---|---|
| Fetal bovine serum | Biotin |
| Pantothenate | Human insulin |
| Dexamethasone | Antibiotics |

**Adipocytes culture**

1. Subcutaneous preadipocytes from Zen-Bio Inc were thawed and plated into appropriate experimental plate format.
2. Upon thawing cells, culturing followed differentiation schedule below:

**Day Treatment**

1 Plate cells in PM-1
2 Differentiate cells using DM-2
9 Feed cells Maintenance medium AM-1
16 Assay cells for lipid accumulation, lipolysis, glucose uptake or RNAseq.

**Adipocyte Glucose Uptake Assay**

1. Primary human adipocytes were differentiated in 96-well isoplates

2. At 14 days post differentiation, the cells were treated with glucose uptake starvation media (Zen-Bio Inc) overnight.

3. The next morning, the cells were washed with assay buffer and the cells were treated with compounds in glucose assay buffer containing $^3$H-2-deoxyglucose cocktail and the cells were incubated at 37°C, 5% $CO_2$ and allowed to incubate for 2 hours.

4. After 2 hours, the cells were washed with PBS, lysed, and mixed with Optiphase "Supermix" scintillation fluid (Zen-Bio Inc). The Counts Per Minute (CPM) of each well measured.

**Controls used to test experimental set up:**

Vehicle control: DMSO (control)
Zen Bio Insulin positive control: 100 nM (insulin)
Control for possible endotoxin contamination of pHMOs: Lipopolysaccharide (LPS) 2ng/mL (LPS).

In this experiment the same pool of HMOs (pHMOs) as in example 2 has been tested in addition to 2'FL. The results show that pHMOs are as effective in upregulating glucose uptake as insulin, the positive control. 2'FL alone is much less effective, and only has a minor effect on increasing glucose uptake which is not significantly different from the vehicle control. The effect observed by pHMOs is not coming from putative endotoxin contamination of the pHMOs as LPS alone did not upregulate glucose uptake at all.

**Claims**

1. A synthetic composition comprising one or more compounds according to formula 1, or a pharmaceutically acceptable salt, solvate or ester thereof, for therapeutic use in the reduction of the cholesterol level wherein the compound of Formula 1 is

$$Y_n \rightarrow \text{D-Gal-}\beta1 \rightarrow 3\text{-}(Y_m \rightarrow)(X_p \rightarrow)\text{D-GlcNAc-}\beta1 \rightarrow 3\text{-D-Gal-}\beta1 \rightarrow 4\text{-}(X_q \rightarrow)\text{-D-Glc} \qquad \text{Formula 1}$$

wherein

X is $\alpha$-L-Fucose ($\alpha$-L-Fuc),
Y is N-acetyl-D-neuraminic acid (Neu5Ac-$\alpha$-2-)
n, m, p, q indicate the number of the monosaccharide residues present in the compound of formula 1 and n is 0 or 1; m is 0 or 1; p is 0 or 1; q is 0 or 1;
and

$$n+m+p+q \geq 1;$$

preferably, wherein p=1 if q=1;
more preferably, wherein n = 1 and m $\leq$ 1;
particularly preferably,
wherein n=0 and m=0 if p+q $\geq$ 1; or if n+m $\geq$ 1 then p+q =0;
wherein the compound of Formula 1 is selected from the group consisting of lactodifucoheaxose II (LNDFHII, CAS# 62258-12-2), sialyllacto-N-tetraose a (LSTa, CAS# 64003-58-5) and disialyllacto-N-tetraose (DSLNT, CAS# 61278-38-4) or a combination thereof.

2. The synthetic composition for use of any of the preceding claims wherein the use is a reduction of tissue cholesterol levels.

3. The synthetic composition for use of any of the preceding claims wherein the composition comprises two or more compounds selected from the group consisting of lactodifucoheaxose II (LNDFHII), sialyllacto-N-tetraose a (LSTa) and disialyllacto-N-tetraose (DSLNT).

4. The synthetic composition for use of any of the preceding claims wherein the composition comprises lactodifucoheaxose II (LNDFHII), sialyllacto-N-tetraose a (LSTa) and disialyllacto-N-tetraose (DSLNT).

5. The synthetic composition for use of any of the preceding claims wherein the composition further comprises fat, protein and carbohydrate, preferably wherein the fat is a mixture of vegetable fat and milk fat, more preferably wherein the milk fat is bovine milk fat.

6. The synthetic composition for use of any of the preceding claims wherein the composition is an infant formula, preferably, wherein the composition is a formula for children having an age selected from the group consisting of 0-6 months, 0-12 months, 6-12 months 12-24 months, 12-36 months and 24-36 months; or wherein the composition is an adult formula.

7. The synthetic composition for use of any of the preceding claims wherein the composition is a food product selected from the group consisting of confectionary products; nutritionally complete food products; desserts, in particular a pudding, yoghurt, custard, vla, ice-cream, or milk-shake; beverages, in particular a fruit juice or milk; breakfasts, such as porridge, cereals; soups; and sauces.

8. The synthetic composition for use of any of the preceding claims wherein the composition is further comprising one or more human milk oligosaccharides (HMOs) other than a compound according to formula 1; preferably, wherein the composition further comprises LNFP II or LNFP III, more preferably, wherein the composition further comprises LNFP II and LNFP III.

9. The synthetic composition for use of any of the preceding claims further comprising one or more probiotics and / or one or more prebiotics.

10. The synthetic composition for use of any of the preceding claims further comprising a biologically effective amount of lactoferrin, preferably comprising more than 0.01 wt% of dry composition.

11. The synthetic composition for use of any of the preceding claims
**characterized in that** the composition does not comprise purified adiponectin or a biologically active fragment thereof.

12. The synthetic composition for use of any of the preceding claims further comprising soluble CD14.

13. The synthetic composition for use of any of the preceding claims wherein the amount of compound according to formula 1 is between 0.001 wt% and 15 wt% of total dry weight of the composition, preferably between 0.01 and 10 wt%, more preferably between 0.01 and 5 wt%.

14. The synthetic composition for use of any of the preceding claims preferably for use in a human.

**Patentansprüche**

1. Synthetische Zusammensetzung, umfassend eine oder mehrere Verbindungen gemäß Formel 1 oder ein pharmazeutisch unbedenkliches Salz, Solvat oder einen pharmazeutisch unbedenklichen Ester davon, zur therapeutischen Verwendung bei der Verringerung des Cholesterinspiegels, wobei die Verbindung der Formel 1

$$Y_n \rightarrow \text{D-Gal-}\beta 1 \rightarrow 3\text{-}(Y_m \rightarrow)(X_p \rightarrow)\text{D-GlcNAc-}\beta 1 \rightarrow 3\text{-D-Gal-}\beta 1 \rightarrow 4\text{-}(X_q \rightarrow)\text{-D-Glc} \qquad \text{Formel 1}$$

ist, worin:

X für $\alpha$-L-Fucose ($\alpha$-L-Fuc) steht,
Y für N-Acetyl-D-neuraminsäure (Neu5Ac-$\alpha$-2-) steht n, m, p, q die Anzahl der in der Verbindung der Formel 1 vorhandenen Monosaccharidreste angeben und n für 0 oder 1 steht, m für 0 oder 1 steht, p für 0 oder 1 steht, q für 0 oder 1 steht
und

$$n+m+p+q \geq 1,$$

wobei bevorzugt p=1, wenn q=1,
wobei stärker bevorzugt n = 1 und m $\leq$ 1,
wobei besonders bevorzugt
n=0 und m=0, wenn p+q $\geq$ 1 oder, wenn n+m $\geq$ 1, dann p+q = 0,
wobei die Verbindung der Formel 1 aus der aus Lactodifucoheaxose II (LNDFHII, CAS-Nr. 62258-12-2), Sialyllacto-N-tetraose a (LSTa, CAS-Nr. 64003-58-5) und Disialyllacto-N-tetraose (DSLNT, CAS-Nr. 61278-38-4) oder einer Kombination davon bestehenden Gruppe ausgewählt ist.

2. Synthetische Zusammensetzung zur Verwendung nach einem der vorhergehenden Ansprüche, wobei die Verwendung eine Verringerung der Gewebe-Cholesterinspiegel ist.

3. Synthetische Zusammensetzung zur Verwendung nach einem der vorhergehenden Ansprüche, wobei die Zusammensetzung zwei oder mehr aus der aus Lactodifucoheaxose II (LNDFHII), Sialyllacto-N-tetraose a (LSTa) und Disialyllacto-N-tetraose (DSLNT) bestehenden Gruppe ausgewählte Verbindungen umfasst.

4. Synthetische Zusammensetzung zur Verwendung nach einem der vorhergehenden Ansprüche, wobei die Zusammensetzung Lactodifucoheaxose II (LNDFHII), Sialyllacto-N-tetraose a (LSTa) und Disialyllacto-N-tetraose (DSLNT) umfasst.

5. Synthetische Zusammensetzung zur Verwendung nach einem der vorhergehenden Ansprüche, wobei die Zusammensetzung ferner Fett, Protein und Kohlenhydrat umfasst, wobei vorzugsweise das Fett ein Gemisch von Pflanzenfett und Milchfett ist, wobei stärker bevorzugt das Milchfett Rinder-Milchfett ist.

6. Synthetische Zusammensetzung zur Verwendung nach einem der vorhergehenden Ansprüche, wobei die Zusammensetzung eine Säuglingsformulierung ist, wobei die Zusammensetzung vorzugsweise eine Formulierung für Kinder mit einem aus 0-6 Monaten, 0-12 Monaten, 6-12 Monaten, 12-24 Monaten, 12-36 Monaten und 24-36 Monaten bestehenden Gruppe ausgewählten Alter ist, oder wobei die Zusammensetzung eine Erwachsenenformulierung ist.

7. Synthetische Zusammensetzung zur Verwendung nach einem der vorhergehenden Ansprüche, wobei die Zusammensetzung ein Nahrungsmittelprodukt ist, das aus der aus Süßwarenprodukten, diätetisch vollständigen Nahrungsmittelprodukten, Desserts, insbesondere einem Pudding, einem Joghurt, einem Custard, einem Vla, einem Eis oder einem Milchshake, Getränken, insbesondere einem Fruchtsaft oder Milch, Frühstücken, wie Porridge, Cerealien, Suppen, und Soßen bestehenden Gruppe ausgewählt ist.

8. Synthetische Zusammensetzung zur Verwendung nach einem der vorhergehenden Ansprüche, wobei die Zusammensetzung ferner ein oder mehrere andere Oligosaccharide aus menschlicher Milch (human milk oligosaccharides, HMO) als eine Verbindung gemäß Formel 1 umfasst, wobei vorzugsweise die Zusammensetzung ferner LNFP II oder LNFP III umfasst, wobei stärker bevorzugt die Zusammensetzung ferner LNFP II und LNFP III umfasst.

9. Synthetische Zusammensetzung zur Verwendung nach einem der vorhergehenden Ansprüche, die ferner ein oder mehrere Probiotika und / oder ein oder mehrere Präbiotika umfasst.

10. Synthetische Zusammensetzung zur Verwendung nach einem der vorhergehenden Ansprüche, die ferner eine biologisch wirksame Menge an Lactoferrin umfasst, vorzugsweise mehr als 0,01 Gew.-%, bezogen auf die trockene Zusammensetzung, umfasst.

11. Synthetische Zusammensetzung zur Verwendung nach einem der vorhergehenden Ansprüche, **dadurch gekennzeichnet, dass** die Zusammensetzung kein gereinigtes Adiponectin oder ein biologisch aktives Fragment davon umfasst.

12. Synthetische Zusammensetzung zur Verwendung nach einem der vorhergehenden Ansprüche, die ferner lösliches CD14 umfasst.

13. Synthetische Zusammensetzung zur Verwendung nach einem der vorhergehenden Ansprüche, wobei die Menge an Verbindung gemäß Formel 1 zwischen 0,001 Gew.-% und 15 Gew.-%, bezogen auf das Gesamt-Trockengewicht der Zusammensetzung, vorzugsweise zwischen 0,01 und 10 Gew.-%, stärker bevorzugt zwischen 0,01 und 5 Gew.-%, beträgt.

14. Synthetische Zusammensetzung zur Verwendung nach einem der vorhergehenden Ansprüche, vorzugsweise zur Verwendung bei einem Menschen.


**Revendications**

1. Composition synthétique comprenant un ou plusieurs composés selon la formule 1, ou un sel, solvate ou ester pharmaceutiquement acceptable de ceux-ci, pour une utilisation thérapeutique dans la réduction du taux de cholestérol, dans laquelle le composé de Formule 1 est

$$Y_n \rightarrow D\text{-}Gal\text{-}\beta1 \rightarrow 3\text{-}(Y_m \rightarrow)(X_p \rightarrow)D\text{-}GlcNAc\text{-}51,3\text{-}D\text{-}Gal\text{-}51,4\text{-}(X_q \rightarrow)\text{-}D\text{-}Glc \qquad \text{Formule 1}$$

dans laquelle

X est $\alpha$-L-Fucose ($\alpha$-L-Fuc),
Y est un acide N-acétyl-D-neuraminique (Neu5Ac-$\alpha$-2-) n, m, p, q indiquent le nombre de résidus monosaccharidiques présents dans le composé de formule 1 et n est 0 ou 1 ; m est 0 ou 1 ; p est 0 ou 1 ; q est 0 ou 1 ; et

$$n+m+p+q \geq 1 \; ;$$

préférablement, dans laquelle p = 1 si q = 1 ;
plus préférablement, dans laquelle n = 1 et m $\leq$ 1 ; particulièrement préférablement,
dans laquelle n = 0 et m = 0 si p+q $\geq$ 1 ; ou si n+m $\geq$ 1 alors p+q = 0 ;
dans laquelle le composé de Formule 1 est choisi dans le groupe constitué du lactodifucohéaxose II (LNDFHII, n° CAS 62258-12-2), du sialyllacto-N-tétraose a (LSTa, n° CAS 64003-58-5) et du disialyllacto-N-tétraose (DSLNT, n° CAS 61278-38-4) ou une combinaison de ceux-ci.

2. Composition synthétique pour utilisation selon l'une quelconque des revendications précédentes, dans laquelle l'utilisation est une réduction des taux de cholestérol tissulaire.

3. Composition synthétique pour utilisation selon l'une quelconque des revendications précédentes, dans laquelle la composition comprend deux ou plusieurs composés choisis dans le groupe constitué du lactodifucohéaxose II (LNDFHII), du sialyllacto-N-tétraose a (LSTa) et du disialyllacto-N-tétraose (DSLNT).

4. Composition synthétique pour utilisation selon l'une quelconque des revendications précédentes, dans laquelle la composition comprend du lactodifucohéaxose II (LNDFHII), du sialyllacto-N-tétraose a (LSTa) et du disialyllacto-N-tétraose (DSLNT).

5. Composition synthétique pour utilisation selon l'une quelconque des revendications précédentes, dans laquelle la composition comprend en outre de la graisse, des protéines et des glucides, préférablement dans laquelle la graisse est un mélange de graisses végétales et de graisse de lait, plus préférablement dans laquelle la graisse de lait est de la graisse de lait de bovin.

6. Composition synthétique pour utilisation selon l'une quelconque des revendications précédentes, dans laquelle la composition est une formule pour nourrissons, préférablement, dans laquelle la composition est une formule pour enfants ayant un âge choisi dans le groupe constitué de 0-6 mois, 0-12 mois, 6-12 mois, 12-24 mois, 12-36 mois et 24-36 mois ; ou dans laquelle la composition est une formule pour adultes.

7. Composition synthétique pour utilisation selon l'une quelconque des revendications précédentes, dans laquelle la composition est un produit alimentaire choisi dans le groupe constitué des produits de confiserie ; des produits alimentaires nutritionnellement complets ; des desserts, en particulier un pudding, un yaourt, une crème anglaise, un vla, une crème glacée ou un milk-shake ; des boissons, en particulier un jus de fruits ou du lait ; des petits-déjeuners, tels que du porridge, des céréales ; des soupes ; et des sauces.

8. Composition synthétique pour utilisation selon l'une quelconque des revendications précédentes, dans laquelle la composition comprend en outre un ou plusieurs oligosaccharides du lait humain (HMO) autres qu'un composé selon la formule 1 ; préférablement, dans laquelle la composition comprend en outre du LNFP II ou du LNFP III, plus préférablement, dans laquelle la composition comprend en outre du LNFP II et du LNFP III.

9. Composition synthétique pour utilisation selon l'une quelconque des revendications précédentes, comprenant en outre un ou plusieurs probiotiques et/ou un ou plusieurs prébiotiques.

10. Composition synthétique pour utilisation selon l'une quelconque des revendications précédentes, comprenant en outre une quantité biologiquement efficace de lactoferrine, comprenant préférablement plus de 0,01 % en poids de composition sèche.

11. Composition synthétique pour utilisation selon l'une quelconque des revendications précédentes, **caractérisée en ce que** la composition ne comprend pas d'adiponectine purifiée ou un fragment biologiquement actif de celle-ci.

12. Composition synthétique pour utilisation selon l'une quelconque des revendications précédentes, comprenant en outre du CD14 soluble.

13. Composition synthétique pour utilisation selon l'une quelconque des revendications précédentes, dans laquelle la quantité de composé selon la formule 1 est comprise entre 0,001 % en poids et 15 % en poids du poids sec total de la composition, préférablement entre 0,01 et 10 % en poids, plus préférablement entre 0,01 et 5 % en poids.

14. Composition synthétique pour utilisation selon l'une quelconque des revendications précédentes, préférablement pour utilisation chez un humain.

Figure 1

**Relative Cholesterol Uptake**

Figure 2

**Glucose Uptake**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019052529 A
- US 20090098240 A
- WO 2012112777 A

**Non-patent literature cited in the description**

- **PARK Y.M.** *Exp Mol Med.*, 06 June 2014, vol. 46, e99
- **NICHOLSON AC**. *Trends Cardiovasc Med.*, January 2004, vol. 14 (1), 8-12
- **RUBIC T** ; **LORENZ RL**. *Cardiovasc Res.*, 01 February 2006, vol. 69 (2), 527-35
- **GABUNIA K** ; **ELLISON S** ; **KELEMEN S** ; **KAKO F** ; **CORNWELL WD** ; **ROGERS TJ** ; **DATTA PK** ; **OUIMET M** ; **MOORE KJ** ; **AUTIERI MV**. *Am J Pathol.*, May 2016, vol. 186 (5), 1361-74
- **OHIRA H** ; **TSUTSUI W** ; **FUJIOKA Y**. *; J Atheroscler Thromb.*, 01 July 2017, vol. 24 (7), 660-672
- **TRIANTIS V** ; **BODE L** ; **VAN NEERVEN RJJ**. *Front Pediatr.*, 02 July 2018, vol. 6, 190
- Advances in Nutrition. *American Society for Nutrition*, May 2012, vol. 3, 4065-4145
- *CHEMICAL ABSTRACTS*, 62258-12-2
- *CHEMICAL ABSTRACTS*, 64003-58-5
- *CHEMICAL ABSTRACTS*, 61278-38-4
- **L. BODE**. *J. Nutr.*, 2006, vol. 136, 2127-2130
- *CHEMICAL ABSTRACTS*, 41263-94-9
- **ADAMS et al.** *Nutrafoods*, 2018, 169-173
- *CHEMICAL ABSTRACTS*, 21973-23-9
- *CHEMICAL ABSTRACTS*, 25541-09-7